# EUROPÄISCHE PATENTSCHRIFT

(11) **EP 3 037 427 B1**
(45) Veröffentlichungstag und Bekanntmachung des Hinweises auf die Patenterteilung: **23.11.2016**
(21) Anmeldenummer: 15172068.7
(22) Anmeldetag: 04.12.2014
(51) Int. Cl.: C07F 9/6574, C07B 41/06, C07C 45/50, B01J 31/28

(54) **MONOPHOSPHITE DIE EIN NAPHTHOL AUFWEISEN**
MONOPHOSPHITES WITH A NAPHTHOL
MONOPHOSPHITES PRÉSENTANT DU NAPHTOL

(43) Veröffentlichungstag der Anmeldung: 29.06.2016
(62) Teilanmeldung aus: 14196194.6
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Hess, Dieter, 45770 Marl (DE); Fridag, Dirk, 45721 Haltern am See (DE); Geilen, Frank, 45721 Haltern am See (DE); Börner, Armin, 18059 Rostock (DE); Selent, Detlef, 18059 Rostock (DE)

(56) Entgegenhaltungen:
- WO-A2-2004/076464
- RO-A1- 96 102
- DATABASE CA [Online] CHEMICAL ABSTRACTS SERVICE, COLUMBUS, OHIO, US; 1967, VERIZHNIKOV, L. V. ET AL: "Synthesis of esters of O,O'-biphenylylenephosphorous acid", XP002739614, gefunden im STN Database accession no. 1968:12597
- MASANORI KAWASAKI ET AL: "Enantioselective O-Nitroso Aldol Reaction of Silyl Enol Ethers", ANGEWANDTE CHEMIE INTERNATIONAL EDITION, Bd. 47, Nr. 20, 5. Mai 2008 (2008-05-05), Seiten 3795-3797, XP055189221, ISSN: 1433-7851, DOI: 10.1002/anie.200705679

## Beschreibung

Die Erfindung betrifft Monophosphite, die ein Naphthol aufweisen. Des Weiteren deren Verwendung als Liganden in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P(III). Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Verbindung 6-(Naphthalin-1-yloxy)dibenzo[d,f][1,3,2]dioxaphosphepin ist bereits bekannt (CAS 16611-80-6) und kann durch die Reaktion von 1-Naphthol und 6-Chlorodibenzo[*d*,*f*][1,3,2]dioxaphosphepin in Gegenwart einer Base hergestellt werden.

WO2004/076464 offenbart Monophosphite auf Basis eines 1,1'-Biphenyl-2,2'-diols und deren Verwendung als Liganden in Metall-Komplexen für Katalysatoren der Hydroformylierung. RO96102 offenbart [1,3,2]dioxaphosphepin-6-yl]oxy][1,2'-binaphthalen]-2-yl-diphenylester als Liganden in Hydroformylierungs-Katalysatoren. MASANORI KAWASAKI ET AL. in ANGEWANDTE CHEMIE INTERNATIONAL EDITION 2008, Vol 47(20), Seiten 3795-3797 offenbaren Binaphthol-basierte Monophosphite als Liganden für enantioselektive Nitroso-Aldol-Reaktionen.

Der Erfindung lag die Aufgabe zugrunde, Monophosphite bereitzustellen, welche gegenüber den bekannten Monophosphiten vorteilhafte Eigenschaften in der Hydroformylierung aufweisen. Insbesondere bestand die Aufgabe darin, neue Liganden bereitzustellen, deren Einsatz gegenüber strukturverwandten Monophosphiten zu einer verbesserten Selektivität führen. Die verbesserte Selektivität sollte bei zumindest einem Olefin realisiert werden.

Die Aufgabe wird gelöst durch eine Verbindung nach Anspruch 1.

Verbindung, welche die allgemeine Struktur **II** aufweist: 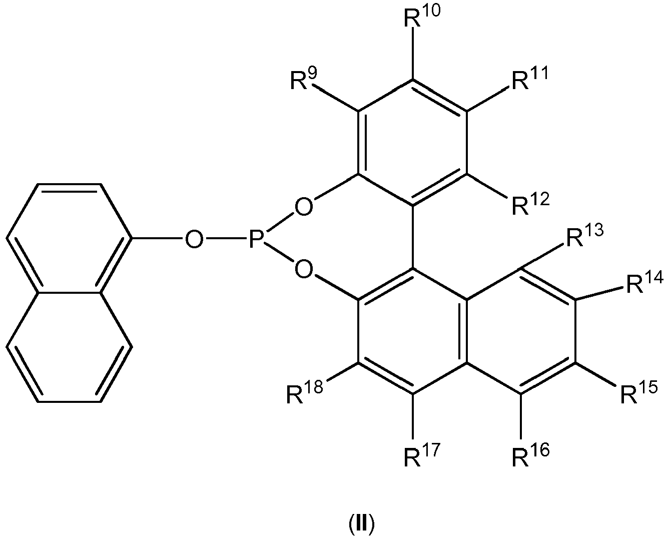 wobei
R⁹, R¹⁰, R¹¹, R¹², R¹³, R¹⁴, R¹⁵, R¹⁶, R¹⁷, R¹⁸ ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H;-SO₃Na, -NO₂, -CN, -NH₂, -N[(C₁-C₁₂)-Alkyl]₂.
(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.
(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Dimethylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, -Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter-(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.

Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter - H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂. Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte-(C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl.

In einer Ausführungsform weist die Verbindung eine der folgenden Strukturen (2) oder (3) auf:

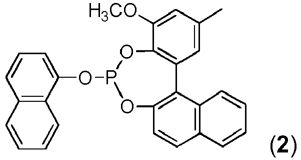

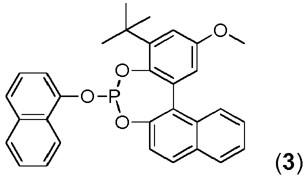

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

In einer bevorzugten Variante des Verfahrens ist das Metallatom Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 200 °C und ein Druck von 1 bar bis 300 bar. Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2-oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Im Folgenden wird die Erfindung anhand von Ausführungsbeispielen näher erläutert.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).
Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Biphenole

Die Synthese der Biphenole erfolgt analog zu DE102013203865 und DE102013203867.

### Synthese der Chlorophosphite

Die Synthese der Monochlorophosphite wie 6-Chlorodibenzo [*d*,*f*][1,3,2]dioxaphosphepin ist dem Fachmann bekannt und erfolgt nach bekannter Weise. Chlorophosphite lassen sich durch Zusatz von Phosphortrichlorit in Gegenwart einer Base herstellen. Für nähere Informationen siehe auch "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.

### Synthese der Monophosphite

### 6-(Naphthalin-1-yloxy)dibenzo[d,f][1,3,2]dioxaphosphepin

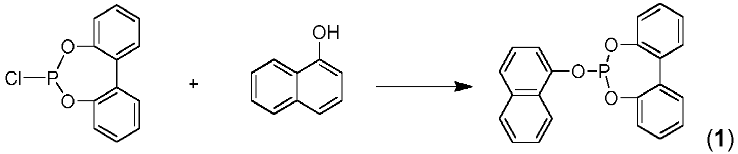

Zu einer auf -20 °C gekühlten Lösung von 1-Naphthol (0,393 g; 2,73 mmol) in THF (8 ml) wird tropfenweise eine Lösung von n-BuLi in Hexan (8,5 ml einer 0,32 M Lösung; 2,73 mmol). Man lässt auf Raumtemperatur erwärmen und gibt zu dieser Mischung eine Lösung von 6-Chlorodibenzo[*d*,*f*][1,3,2]dioxaphosphepin (0,684 g; 2,73 mmol) in THF (5 ml). Man rührt über Nacht, filtriert und engt das Filtrat zur Trockne ein. Der Rückstand wird durch Säulenchromatografie gereinigt (Hexan/Toluol, 1:1, R*_{f}*= 0,5). Ausbeute: 0,61 g (1,7 mmol; 63%). ³¹P-NMR (CD₂Cl₂): 143,8 ppm.

### 4-Methoxy-2-methyl-6-(naphthalen-1-yloxy)benzo[d]naphtho[1,2-f][1,3,2]dioxaphosphepin

### a) Vorstufe, N,N-Diethyl-2-methoxy-4-methylbenzo[d]naphtho[1,2-f][1,3,2]dioxaphosphepin-6-amin

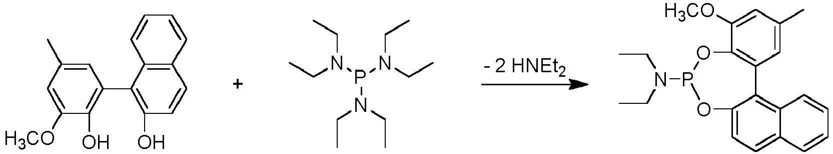

Eine Lösung von 1-(2-Hydroxy-3-methoxy-5-methylphenyl)naphthalen-2-ol (0,661 g; 2,36 mmol) in Toluol (10 ml) wurde mit Tris(diethylamino)phosphin (0,583 g; 2,36 mmol) versetzt. Die Reaktionslösung wurde für 20 h bei Raumtemperatur gerührt. Das Lösungsmittel wurde im Vakuum entfernt und der Rückstand getrocknet. Ausbeute: 0,819 g (2,147 mmol; 90 %). Elementaranalyse (berechnet für C₂₂H₂₄NO₃P = 381,40 g/mol) C 68,69 (68,47); H 6,19 (6,29); N 3,79 (3,80); P 8,45 (8,41)%.
³¹P-NMR (CD₂Cl₂): 149,2; 150,8 ppm.
EI-MS: m/e 381 [M⁺] (28%); 366 [M-CH₃]⁺ (43%); 309 [M-N(C₂H₅)]⁺ (100%).

### b) 1-Naphthylphosphit

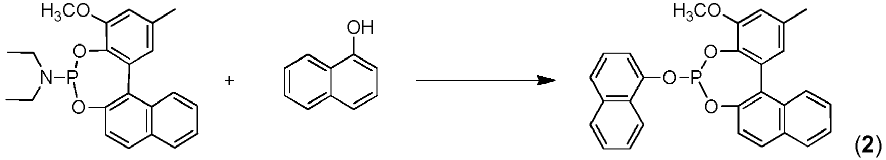

Zu einer gerührten Lösung von *N*,*N*-Diethyl-4-methoxy-2-methylbenzo[*d*]naphtho[1,2-*f*][1,3,2]dioxaphosphepin-6-amin (0,749 g; 1,96 mmol) in Toluol (10 ml)wurde 1-Naphthol (0,577 g; 3,92 mmol) gegeben. Das Reaktionsgemisch wurde für 69 h bei 110 °C gerührt. Das Lösungsmittel wurde im Vakuum entfernt und der erhaltene Rückstand bei 70 °C / 0,1 mbar getrocknet. Das Rohprodukt wurde durch Säulenchromatografie gereinigt (Dichlormethan, R*_{f}* = 0,94). Ausbeute: 0,540 g (1,194 mmol; 60%).

Elementaranalyse (berechnet für C₂₈H₂₁O₄P = 452,4 g/mol) C 73,93 (74,33); H 5,06 (4,68); P (6,85)%.
³¹P-NMR (CD₂Cl₂): 141,8; 146,4 ppm.
¹H-NMR (CD₂Cl₂): 2,48+2,52 (2s, 3 H); 3,82+4,04 (2s, 3 H); 6,92-8,37 (m, 15 H, Hₐᵣₒₘ) ppm.
¹³C-NMR (CD₂Cl₂): 21,7; 21,8; 56,0; 56,6; 112,8; 113,3; 114,8; 115,0; 115,4; 115,6; 121,2; 121,9; 122,5; 122,6; 124,4; 124,5; 124,6; 125,5; 125,7; 125,9; 126,0; 126,0; 126,4; 126,5; 127,0; 127,1; 127,2; 127,3; 127,8; 128,0; 128,8; 128,8; 129,0; 129,9; 130,4; 132,2; 132,4; 132,6; 134,8; 135,2; 135,3; 135,5; 146,2; 147,1; 148,0; 148,1; 148,2; 152,1; 152,5 ppm.

### 4-(tert-Butyl)-2-methoxy-6-(naphthalen-1-yloxy)benzo[d]naphtho[1,2-f][1,3,2]dioxaphosphepin

### a) Vorstufe, 4-(tert-Butyl)-N,N-diethyl-2-methoxybenzo[d]naphtho[1,2-f][1,3,2]dioxaphosphepin-6-amin

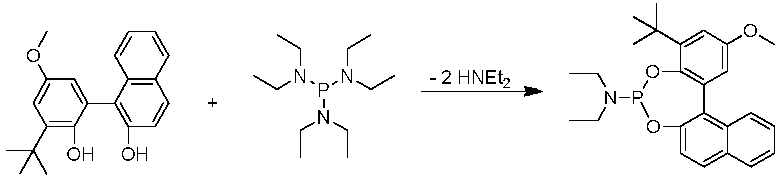

Eine Lösung von 1-(3-(*tert*-Butyl)-2-hydroxy-5-methoxyphenyl)naphthalen-2-ol (0,675 g; 2,09 mmol) in Toluol (10ml) wurde mit Tris(diethylamino)phosphin (0,517 g; 2,09 mmol) versetzt. Die Reaktionslösung wurde für 62 h bei Raumtemperatur und für 2h bei 70 °C gerührt. Das Lösungsmittel wurde im Vakuum entfernt und der Rückstand getrocknet. Ausbeute: 0,820 g (1,936 mmol; 92 %).
Elementaranalyse (berechnet für C₂₅H₃₀NO₃P = 423,5 g/mol) C 70,98 (70,90); H 7,28 (7,14); N 3,22 (3,31); P 7,36 (7,31)%.
³¹P-NMR (CD₂Cl₂): 149,6 ppm.

### b) 1-Naphthylphosphit

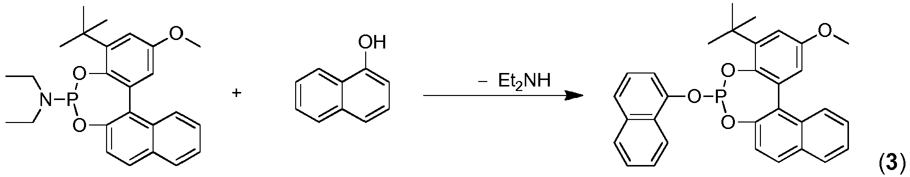

Zu einer gerührten Lösung von 4-(*tert*-Butyl)-*N,N*-diethyl-2-methoxybenzo[*d*]naphtho[1,2-*f*][1,3,2]dioxaphosphepin-6-amin (0,783 g; 1,849 mmol) in Toluol (10 ml) wurde 1-Naphthol (0,533 g; 3,697 mmol) gegeben. Das Reaktionsgemisch wurde für 69 h bei 110 °C gerührt. Das Lösungsmittel wurde im Vakuum entfernt und der erhaltene Rückstand bei 70 °C / 0,1 mbar getrocknet. Das Rohprodukt wurde durch Säulenchromatografie gereinigt (Hexan/Essigsäureethylester, 8:1, R*_{f}* = 0,38). Ausbeute: 0,430 g (0,870 mmol; 47%). Elementaranalyse (berechnet für C₃₁H₂₇O₄P = 494,52 g/mol) C 74,48 (75,29); H 5,71 (5,50); P (6,26) %.
³¹P-NMR (CD₂Cl₂): 144,5 ppm.
¹H-NMR (CD₂Cl₂): 1,59 (s, 9 H); 3,86 (s, 3 H); 7,04 (m, 1 H); 7,16 (m, 1 H,); 7,37-7,51 (m, 3 H); 7,51-7,62 (m, 4 H); 7,68-7,75 (m, 1 H); 7,88-8,00 (m, 3 H); 8,07-8,22 (m, 2 H) ppm.
¹³C-NMR (CD₂Cl₂): 31,3; 31,3; 35,8; 56,0; 115,0; 115,1; 115,2; 115,3; 121,6; 122,4; 124,7; 125,5; 125,9; 125,9; 126,0; 126,6; 127,1; 127,2; 127,7; 128,1; 128,9; 129,9; 130,6; 130,6; 132,3; 132,8; 135,3; 141,9; 144,2; 144,3; 146,6; 146,7; 148,1; 148,2; 156,0 ppm.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Als Olefin wurde ein Octengemisch eingesetzt: n-Octene (Oxeno GmbH, Octenisomerengemisch aus 1-Octen: ∼3 %; *cis+trans-*2-Octen: ∼49%; *cis*+*trans-*3-Octen: ∼29%; *cis*+*trans*-Octen-4: ∼16%; gerüstisomere Octene: ∼3 % wurden mehrere Stunden über Natrium am Rückfluß erhitzt und unter Argon destilliert.
Für die Versuche wurden im Autoklaven unter Argonatmosphäre folgende Lösungen des Rhodiums in Form von [(acac)Rh(COD)] (acac = Acetylacetonat-Anion; COD = 1,5-Cyclooctadien, Umicore) als Katalysatorvorstufe in Toluol eingefüllt: Für Versuche mit 100 ppm-m Rhodium 10 ml einer 4,31 millimolaren, für 40 bzw. 60 ppm-m die gleiche Menge einer entsprechend verdünnten Lösung. Anschließend wurde die entsprechende Menge der in Toluol gelösten Phosphitverbindung (5 Ligandäquivalente pro Rhodium) zugemischt. Durch Zugabe von weiterem Toluol (Die Gesamtmasse an Toluol wurde bestimmt für die GC-Analyse, s.u.) wurde das Anfangsvolumen der Katalysatorlösung auf 41,0 ml eingestellt. Die jeweils eingebrachte Masse an Toluol wurde bestimmt. Einwaage n-Octene: 10,70 g (95,35 mmol). DerAutoklav wurde bei einem Gesamtgasdruck (Synthesegas: Linde; H₂ (99,999%): CO (99,997%) = 1:1) von a) 42 bar für einen Enddruck von 50 bar bzw. b) 12 bar für den Enddruck von 20 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar bzw. b) 19,5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Katalyseergebnisse

Die Ergebnisse der Katalyseversuche sind in der Tabellen 1 zusammengefasst.

**Tabelle 1:**

| **Ligand** | **p (bar)** | **T (°C)** | **t (h)** | **[Rh] (ppm)** | **L/Rh** | **Ausb. (%)** | **n-Sel. (%)** |
|---|---|---|---|---|---|---|---|
| **1** | 20 | 120 | 4 | 100 | 5 | 90 | 24,9 |
| **2*** | 20 | 120 | 4 | 100 | 5 | 89 | 27,7 |
| **3*** | 20 | 120 | 4 | 100 | 5 | 90 | 33,5 |

| | | | | | | | |
|---|---|---|---|---|---|---|---|
| * erfindungsgemäße Verbindung Olefin: n-Octene (Oxeno GmbH) Solvens: Toluol Verhältnis Ligand/Rhodium (L/Rh): 5 : 1 | | | | | | | |

Alle erfindungsgemäßen Liganden weißen eine höhere Selektivität auf, als der Vergleichsligand (1).

Anhand der oben beschriebenen Versuche konnte gezeigt werden, dass die gestellte Aufgabe durch die erfindungsgemäßen Verbindungen gelöst wird.

## Patentansprüche

1. Verbindung, welche die allgemeine Struktur **II** aufweist: 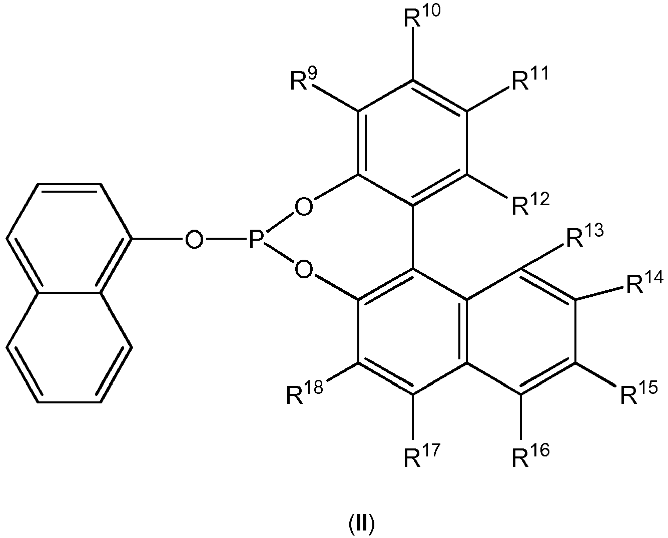 wobei
R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl,-(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; - SO₃Na, -NO₂, -CN, -NH₂, -N[(C₁-C₁₂)-Alkyl]₂.

2. Verbindung nach Anspruch 1,
wobei R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

3. Verbindung nach einem der Ansprüche 1 oder 2,
wobei R⁹, R¹⁰, R¹¹, R¹², R¹³, R¹⁴, R¹⁵, R¹⁶, R¹⁷, R¹⁸ ausgewählt sind aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

4. Verbindung nach einem der Ansprüche 1 bis 3,
wobei R⁹, R¹⁰, R¹¹, R¹², R¹³, R¹⁴, R¹⁵, R¹⁶, R¹⁷, R¹⁸ ausgewählt sind aus:
- H, -(C₁-C₁₂)-Alkyl, -O-(C₁-C₁₂)-Alkyl, -O-(C₆-C₂₀)-Aryl.

5. Verbindung nach einem der Ansprüche 1 bis 4,
wobei R⁹, R¹⁰, R¹¹, R¹², R¹³, R¹⁴, R¹⁵, R¹⁶, R¹⁷, R¹⁸ ausgewählt sind aus:
- H, -(C₁-C₁₂)-Alkyl, -O-(C₁-C₁₂)-Alkyl.

6. Verbindung nach einem der Ansprüche 1 bis 5,
welche eine der folgenden Strukturen (2) oder (3) aufweist:

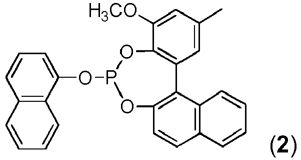

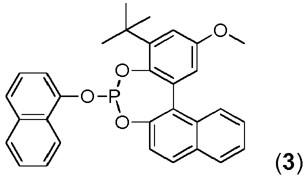

7. Komplex umfassend:
- eine Verbindung nach einem der Ansprüche 1 bis 6,
- ein Metallatom ausgewählt aus: Rh, Ru, Co, Ir.

8. Verwendung einer Verbindung nach einem der Ansprüche 1 bis 6,
zur Katalyse einer Hydroformylierungsreaktion.

9. Verfahren umfassend die Verfahrensschritte:
a) Vorlegen eines Olefins,
b) Zugabe eines Komplexes nach Anspruch 7,
oder einer Verbindung nach einem der Ansprüche 1 bis 6 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having the general structure II: 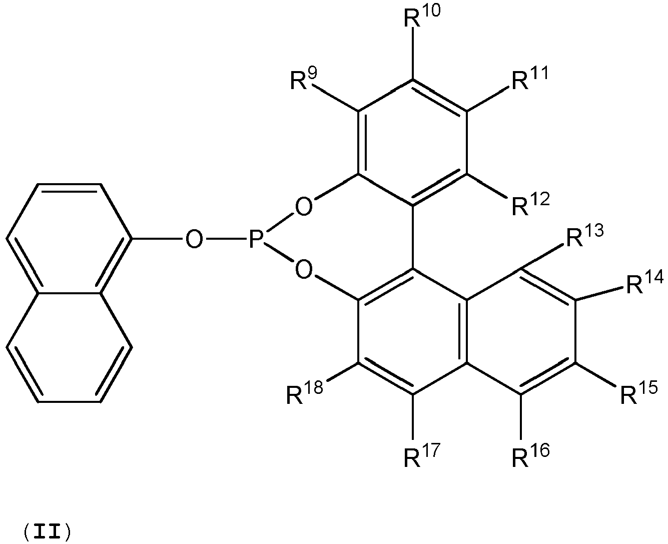 where
R⁹, R¹⁰, R¹¹, R¹², R¹³, R¹⁴, R¹⁵, R¹⁶, R¹⁷, R¹⁸ are selected from:
- H, -C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, COO-(C₁-C₁₂)-alkyl, -CONH-(C₁-C₁₂)-alkyl, -CO-(C₁-C₁₂)-alkyl, -CO-(C₆-C₂₀)-aryl, -COOH, -OH, -SO₃H, -NH₂, -N[(C₁-C₁₂)-alkyl]₂; where the alkyl and aryl groups mentioned may be substituted as follows:
substituted -(C₁-C₁₂)-alkyl groups and substituted -(C₁-C₁₂)-alkoxy groups may have one or more substituents, depending on their chain length; the substituents are each independently selected from -(C₃-C₁₂)-cycloalkyl,-(C₃-C₁₂) -heterocycloalkyl, - (C₆-C₂₀) -aryl, fluorine, chlorine, cyano, formyl, acyl and alkoxycarbonyl;
substituted - (C₆-C₂₀) -aryl groups and -(C₆-C₂₀)-aryl- (C₆-C₂₀)-aryl groups may have one or more substituents, depending on the ring size; these substituents are each independently selected from -H, -(C₁-C₁₂)-alkyl, -O-(C₁-C₁₂)-alkyl, -O-(C₆-C₂₀)-aryl, -(C₆-C₂₀)-aryl, -halogen,-COO-(C₁-C₁₂)-alkyl, -CONH-(C₁-C₁₂)-alkyl, -(C₆-C₂₀-aryl-CON[(C1-C12)-alkyl]₂, -CO-(C₁-C₁₂)-alkyl, -CO-(C₆-C₂₀)-aryl, -COOH, -OH, -SO₃H, -SO₃Na, -NO₂, -CN, -NH₂, -N[(C₁-C₁₂)-alkyl]₂.

2. Compound according to Claim 1,
where R⁹, R¹⁰, R¹¹, R¹², R¹³, R¹⁴, R¹⁵, R¹⁶, R¹⁷, R¹⁸ are selected from:
- H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, -CO-(C₁-C₁₂)-alkyl, -CO-(C₆-C₂₀)-aryl, -N [(C₁-C₁₂)-alkyl]₂.

3. Compound according to either of Claims 1 and 2,
where R⁹, R¹⁰, R¹¹, R¹², R¹³, R¹⁴, R¹⁵, R¹⁶, R¹⁷, R¹⁸ are selected from:
- H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen.

4. Compound according to any of Claims 1 to 3,
where R⁹, R¹⁰, R¹¹, R¹², R¹³, R¹⁴, R¹⁵, R¹⁶, R¹⁷, R¹⁸ are selected from:
- H, -(C₁-C₁₂)-alkyl, -O-(C₁-C₁₂)-alkyl, -O-(C₆-C₂₀)-aryl.

5. Compound according to any of Claims 1 to 4,
where R⁹, R¹⁰, R¹¹, R¹², R¹³, R¹⁴, R¹⁵, R¹⁶, R¹⁷, R¹⁸ are selected from:
- H, -(C₁-C₁₂)-alkyl, -O-(C₁-C₁₂)-alkyl .

6. Compound according to any of Claims 1 to 5, having one of the following structures (2) or (3):

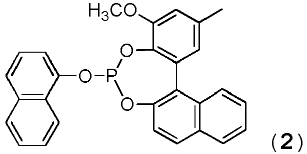

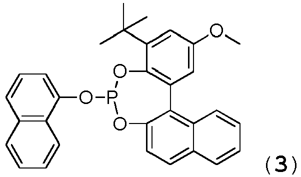

7. Complex comprising:
- a compound according to any of Claims 1 to 6,
- a metal atom selected from: Rh, Ru, Co, Ir,

8. Use of a compound according to any of Claims 1 to 6 for catalysis of a hydroformylation reaction.

9. Process comprising the following process steps:
a) initially charging an olefin,
b) adding a complex according to Claim 7,
or a compound according to any of Claims 1 to 6 and a substance including a metal atom selected from: Rh, Ru, Co, Ir,
c) feeding in H₂ and CO,
d) heating the reaction mixture, with conversion of the olefin to an aldehyde.

## Revendications

1. Composé, qui présente la structure générale **II** : 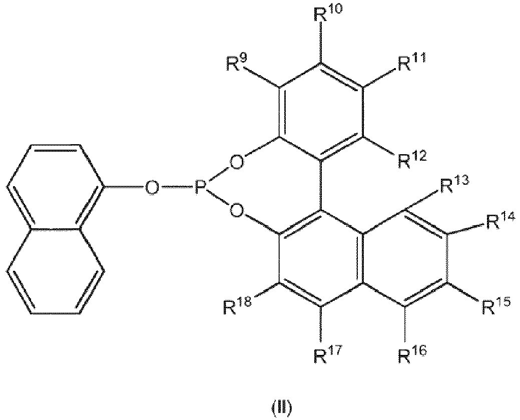 dans laquelle
R⁹, R¹⁰, R¹¹, R¹², R¹³, R¹⁴, R¹⁵, R¹⁶, R¹⁷, R¹⁸ sont choisis parmi :
- H, -alkyle(C₁-C₁₂), -O-alkyle(C₁-C₁₂), -O-aryle (C₆-C₂₀), -aryle(C₆-C₂₀), -S-alkyle, -S-aryle, des atomes d'halogène, -COO-alkyle(C₁-C₁₂), -CONH-alkyle(C₁-C₁₂), -CO-alkyle(C₁-C₁₂), -CO-aryle(C₆-C₂₀), -COOH, -OH, -SO₃H, -NH₂, -N[alkyle(C₁-C₁₂)]₂ ;
les groupes alkyle et aryle nommés pouvant être substitués comme suit :
les groupes -alkyle(C₁-C₁₂) substitués et les groupes -alcoxy(C₁-C₁₂) substitués peuvent comporter, en fonction de leur longueur de chaîne, un ou plusieurs substituants ; les substituants sont choisis, indépendamment les uns des autres, parmi -cycloalkyle(C₃-C₁₂), -hétérocycloalkyle(C₃-C₁₂), -aryle(C₆-C₂₀), fluoro, chloro, cyano, formyle, acyle ou alcoxycarbonyle ;
les groupes -aryle(C₆-C₂₀) substitués et les groupes
- aryl(C₆-C₂₀)-aryle(C₆-C₂₀) substitués peuvent comporter, en fonction de la taille du cycle, un ou plusieurs substituants ; ces substituants sont choisis, indépendamment les uns des autres, parmi -H, -alkyle(C₁-C₁₂), -O-alkyle (C₁-C₁₂) , -O-aryle (C₆-C₂₀), -aryle(C₆-C₂₀), halogéno, -COO-alkyle (C₁-C₁₂), -CONH-alkyle(C₁-C₁₂), -aryl(C₆-C₂₀) -CON [alkyle (C₁-C₁₂)]₂, -CO-alkyle(C₁-C₁₂), -CO-aryle (C₆-C₂₀), -COOH, -OH, -SO₃H, -SO₃Na, -NO₂, -CN, -NH₂, -N [alkyle (C₁-C₁₂)]₂.

2. Composé selon la revendication 1,
dans lequel R⁹, R¹⁰, R¹¹, R¹², R¹³, R¹⁴, R¹⁵, R¹⁶, R¹⁷, R¹⁸ sont choisis parmi :
- H, -alkyle(C₁-C₁₂), -O-alkyle(C₁-C₁₂), -O-aryle(C₆-C₂₀), -aryle(C₆-C₂₀), -S-alkyle, -S-aryle, des atomes d'halogène, -CO-alkyle (C₁-C₁₂), -CO-aryle (C₆-C₂₀), -N[alkyle(C₁-C₁₂)]₂.

3. Composé selon l'une quelconque des revendications 1 et 2,
dans lequel R⁹, R¹⁰, R¹¹, R¹², R¹³, R¹⁴, R¹⁵, R¹⁶, R¹⁷, R¹⁸ sont choisis parmi :
- H, -alkyle(C₁-C₁₂), -O-alkyle(C₁-C₁₂), -O-aryle(C₆-C₂₀), -aryle(C₆-C₂₀), -S-alkyle, -S-aryle, des atomes d'halogène.

4. Composé selon l'une quelconque des revendications 1 à 3,
dans lequel R⁹, R¹⁰, R¹¹, R¹², R¹³, R¹⁴, R¹⁵, R¹⁶, R¹⁷, R¹⁸ sont choisis parmi :
- H, -alkyle(C₁-C₁₂), -O-alkyle (C₁-C₁₂), -O-aryle (C₆-C₂₀).

5. Composé selon l'une quelconque des revendications 1 à 4,
dans lequel R⁹, R¹⁰, R¹¹, R¹², R¹³, R¹⁴, R¹⁵, R¹⁶, R¹⁷, R¹⁸ sont choisis parmi :
- H, -alkyle(C₁-C₁₂), -O-alkyle (C₁-C₁₂).

6. Composé selon l'une quelconque des revendications 1 à 5,
qui présente l'une des structures (2) ou (3) suivantes :

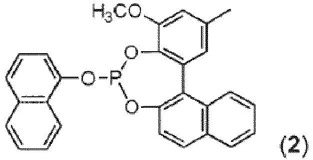

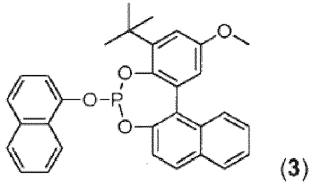

7. Complexe comprenant
- un composé selon l'une quelconque des revendications 1 à 6,
- un atome métallique choisi parmi : Rh, Ru, Co, Ir.

8. Utilisation d'un composé selon l'une quelconque des revendications 1 à 6,
pour la catalyse d'une réaction d'hydroformylation.

9. Procédé comprenant les étapes de processus :
a) disposition au préalable d'une oléfine,
b) addition d'un complexe selon la revendication 7,
ou d'un composé selon l'une quelconque des revendications 1 à 6 et d'une substance qui comporte un atome métallique choisi parmi : Rh, Ru, Co, Ir,
c) introduction de H₂ et CO,
d) chauffage du mélange réactionnel, de sorte que l'oléfine est convertie en un aldéhyde.
